# EUROPEAN PATENT SPECIFICATION

(11) **EP 0 436 499 B1**
(45) Date of publication and mention of the grant of the patent: **09.10.1996**
(21) Application number: 91102716.7
(22) Date of filing: 08.07.1988
(51) Int. Cl.: C01B 25/32

(54) **A process for producing a calcium phosphate type powder**
Verfahren zur Herstellung eines kalziumphosphatartigen Pulvers
Procédé pour la production d'une poudre du type phosphate de calcium

(30) Priority: 10.07.1987 JP 172445/87; 10.07.1987 JP 172446/87; 13.10.1987 JP 257939/87; 13.10.1987 JP 257940/87; 10.06.1988 JP 143122/88
(43) Date of publication of application: 10.07.1991
(62) Divisional of application: 88110950.8
(73) Proprietor: ASAHI KOGAKU KOGYO KABUSHIKI KAISHA, Tokyo (JP)
(72) Inventor: Sumita, Masaya, c/o Asahi Kogaku Kogyo K.K., Itabashi-ku, Tokyo (JP)
(74) Representative: Hansen, Bernd, Dr. Dipl.-Chem.

(56) References cited:
- CHEMICAL ABSTRACTS, vol. 79, no. 20, November 19, 1973, Columbus, Ohio, US; abstract no. 121355J, T.KUTTY, 'Thermal decomposition of hydroxylapatite '

## Description

### FIELD OF THE INVENTION

The present invention relates to a process for producing a composition for forming calcium phosphate type hardening material which is useful as a medical or dental cement material or a bone prosthetic material.

### BACKGROUND OF THE INVENTION

Calcium phosphate compounds, in particular hydroxyapatite, have an excellent biocompatibility and their use as biomaterials in medical or dental fields has been widely investigated.

Calcium phosphate compounds other than apatite are known to be converted to hydroxyapatite upon hydrolysis, and it has recently been found that the hydroxyapatite so produced can be hardened under certain conditions. In an attempt to exploit this phenomenon, several efforts have been made to use calcium phosphate powders as dental or medical cement materials (as described, for example, in Japanese Patent Application (OPI) Nos. 12705/87, 161206/87, 182263/84 and 88351/84) (the term "OPI" as used herein means an unexamined published Japanese patent application)

Calcium phosphate type hardening materials are currently made from three types of calcium phosphate, i.e., α-tricalcium phosphate (Ca₃(PO₄)₂), tetracalcium phosphate (Ca₄(PO₄)₂),and mixtures of these two compounds. In order to prepare α-tricalcium phosphate, calcium carbonate is mixed with calcium pyrophosphate and the mixture is calcined at 1,250°C. To prepare tetracalcium phosphate, calcium carbonate and calcium pyrophosphate are finely ground and mixed, followed by the mixture being calcined at 1,500°C. A mixture of α-tricalcium phosphate and tetracalcium phosphate is prepared by finely grinding and mixing the respective ingredients prepared by the above-described methods.

Since these methods involve a solid phase reaction, the powders obtained lack uniformity in composition. With such powders, a hardening reaction therefore does not proceed uniformly and this results in a hardening material having a reduced strength. Furthermore, because of the high firing temperatures employed, the powders prepared by these conventional methods have low activity and suffer from such disadvantages as a prolonged hardening time and a decrease in the strength of the resulting hardening materials on account of incomplete hardening.

Calcium phosphate powders are hardened by mixing them with a hardening solution. In order to ensure that the hardening material will do no harm to body tissues, it is desirable for the mixture to have a low pH and that the hardening reaction will proceed in the neutral range. An aqueous solution of citric acid has heretofore been considered the best hardening solution capable of satisfying these requirements. However, such an aqueous solution of citric acid still leaves room for improvement, especially when compared with acrylic cement materials which have been commonly used in the art. The considerable problem is that a mixture of a calcium phosphate powder and an aqueous citric acid solution is not highly plastic and cannot be readily formed into a desired shape.

Indian J. Chem. 1973, 11(7), pages 695-697 discloses that heating hydroxylapatite (Ca₅ (PO₄)₃(OH)) at 1250°C in a current of dry air decomposes it to a mixture of Ca₃(PO₄)₂ and Ca₄P₂O₉.

### SUMMARY OF THE INVENTION

An object of the invention is to provide a process for preparing an improved composition for forming a calcium phosphate type hardening material.

This composition allows a hardening reaction to proceed uniformly and thoroughly in the neutral range, can be readily shaped into a desired form by kneading, and produces a hardening material which has high strength and yet does not harm body tissues.

Other objects and effects of the present invention will be apparent from the following description.

The present invention provides a process for producing a calcium phosphate type powder comprising the step of calcining, at a temperature of from about 1,150°C to 1,450°C under reduced pressure, a hydroxyapatite having a molar ratio of Ca/P of about 1.8 or less and more than 1.5 so as to produce a mixture of α-tricalcium phosphate and tetracalcium phosphate.

### BRIEF DESCRIPTION OF THE DRAWINGS

Fig. 1 is a X-ray diffraction scan of the hydroxyapatite synthesized in Example 2; and
Fig. 2 is a X-ray diffraction scan of the powder prepared in Example 2 in accordance with the present invention.

### DETAILED DESCRIPTION OF THE INVENTION

In the process of the present invention, the hydroxyapatite having a molar ratio of Ca/P of more than 1.5 but not exceeding about 1.8 can be readily synthesized by a wet method. If such hydroxyapatite is calcined under reduced pressure at about 1,150°C or more, it is thermally decomposed to form α-tricalcium phosphate and tetracalcium phosphate in admixture. The resulting mixture is uniform in composition. Hydroxyapatite generally starts to be thermally decomposed at a temperature near 1,400°C if the pressure is atmospheric. In the method described above, the pressure is subatmospheric, so the reaction of thermal decomposition is allowed to proceed at a fairly high speed even at the comparatively low temperature of about 1,150°C. This is beneficial to the purpose of simplifying the manufacturing steps and reducing the production cost. The low calcining temperature offers the additional advantage of providing a highly active powder for use as a component in calcium phosphate type hardening materials.

The hydroxyapatite used as the starting material in the process of the invention can be readily synthesized by a wet process in which an aqueous solution of phosphoric acid is reacted with a suspension of calcium hydroxide by a known method. The molar ratio of Ca/P of this hydroxyapatite must be more than 1.5 and about 1.8 or less, preferably from about 1.6 to 1.8. If the molar ratio of Ca/P is 1.5, the product thus-obtained does not become a mixture but α-tricalcium phosphate per se. If the molar ratio of Ca/P exceeds about 1.8, calcium oxide which is deleterious to the human body will form during calcining. The Ca/P molar ratio of the hydroxyapatite used as the starting material can be adjusted by changing the proportions of calcium hydroxide and phosphoric acid which are to be reacted in the synthesis process of the hydroxyapatite. By changing the Ca/P molar ratio of the starting hydroxyapatite, the proportions of α-tricalcium phosphate and tetracalcium phosphate to be finally produced can be adjusted to desired values.

After synthesis by a suitable method such as a wet process, the starting hydroxyapatite is preferably rendered in powder form by suitable means such as filtration, centrifugation or spray-drying. It is also preferred for the hydroxyapatite to be thoroughly dried to remove as much water as possibly by a suitable method such as precalcination at a temperature of about from 500 to 700°C before it is thermally decomposed in a subsequent calcining step.

The thus prepared hydroxyapatite is calcined under reduced pressure at a temperature of from about 1,150°C to 1,450°C. If the calcining temperature is less than about 1,150°C, the intended thermal decomposition reaction will not take place to a practically acceptable extent even if the pressure is decreased.

The reason why the hydroxyapatite is calcined under reduced pressure is that the calcinating temperature can be lowered so as to simplify the manufacturing steps and to reduce the production costs, and that a product of high activity can be attained. The reduced pressure under which the hydroxyapatite is calcined is preferably about 10 Pa or less, more preferably about 10⁻² Pa or less.

The mixture of α-tricalcium phosphate and tetracalcium phosphate which is prepared by thermal decomposition under reduced pressure of the hydroxyapatite having a Ca/P molar ratio of more than 1.5 but not exceeding about 1.8 can be used as a powder component of a composition for producing a hardening material. Additionally, this mixture may be used as a powder for thermal spray as described for example, in Kinzoku Hyomen Gijuttsu Binran (Handbook of Metal Surface Treating Technology), pp. 1,132 to 1,139 (Nikkan Kogyo Shinbunsha, 1963) and Japanese Patent Application (OPI) No. 65871/88. If desired, the powder of the mixture may be granulated and fired at a temperature not higher than the point where conversion to hydroxyapatite takes place, thereby producing granules of bone prosthetic filler. These granules have the advantage that they can be placed at will in any areas of any shape that need prosthetic treatments. Other applications of the mixture include use as a column packing similar to the case of the hydroxyapatite.

The aqueous acidic solution for use as a hardening solution may contain various inorganic and organic acids dissolved therein. Examples thereof include inorganic acids such as phosphoric acid, and organic acids such as acetic acid, lactic acid, citric acid, malic acid, malonic acid, succinic acid, glutaric acid, tartaric acid and polyacrylic acid. These acids are used in an aqueous solution having acid concentrations which are preferably about 25 wt% or more, more preferably from about 25 to 55 wt%. If the acid concentration of the aqueous acidic solution is less than about 25 wt%, the hardening material obtained by mixing it with the powder component will not exhibit the desired strength.

Polysaccharides are added and dissolved in the various aqueous acidic solutions described above. Any polysaccharides can be used if they are soluble in the solution and will not harm body tissues. Examples thereof include chitosan and starch, with chitosan being particularly preferred. The polysaccharides are dissolved in the aqueous acidic solutions in concentrations preferably about 0.05 wt% or more. The effectiveness of the polysaccharides is not sufficient if they are added in concentrations of less than about 0.05wt%.

By using the polysaccharide-containing aqueous acidic solutions as hardening solutions, the α-tricalcium phosphate and tetracalcium phosphate as the powder component can be hardened mildly in the neutral range, and the composition for forming hardening materials is plastic enough to be readily formed into any complex shape.

The hardening reaction can be allowed to proceed even more mildly by further adding and dissolving at least one of glycerin, a monosaccharide, an oligosaccaride and a sugaralcohol in the aqueous acidic solution as the hardening solution having a polysaccharide dissolved therein. Examples of the monosaccharide include glucose and fructose, which may be used either alone or in admixture. Examples of the oligosaccharide include saccharose, maltose, lactose and raffinose, which may be used either alone or in admixture. Examples of the sugaralcohol include sorbitol, mannitol and xylitol, which may also be used either alone or in combination. Glycerin, monosaccharides, oligosaccharides and sugaralcohols may be used either independently or in combination.

When one or more of glycerin, monosaccharides, oligosaccharides and sugaralcohols are used, the total concentration thereof is preferably about 40 wt% or less, more preferably from about 5 to 40 wt%. If the concentration of these additives exceeds about 40 wt%, increased difficulty will be encountered in dissolving them in the aqueous acidic solution.

The powder component and the hardening solution prepared by the procedures described above are mixed so as to initiate the hydrolysis reaction of α-tricalcium phosphate and tetracalcium phosphate, thereby producing hydroxyapatite to provide a hardening material. The powder component and the hardening solution are preferably mixed in such proportions that the ratio of the powder to the solution is within the range of from about 0.4 to 2.7 by weight more preferably from about 0.4 to 2.0 by weight. If the ratio of the powder to the solution is less than about 0.4, the solids content is too low to ensure desired strength for the hardened material. If the ratio of the powder to the solution exceeds about 2.7, it becomes difficult to attain uniform mixing of the powder and the solution.

The following examples are provided for the purpose of further illustration the present invention but are in no way to be taken as limiting. All parts, ratio and presents are by weight unless otherwise indicate.

### EXAMPLE 1

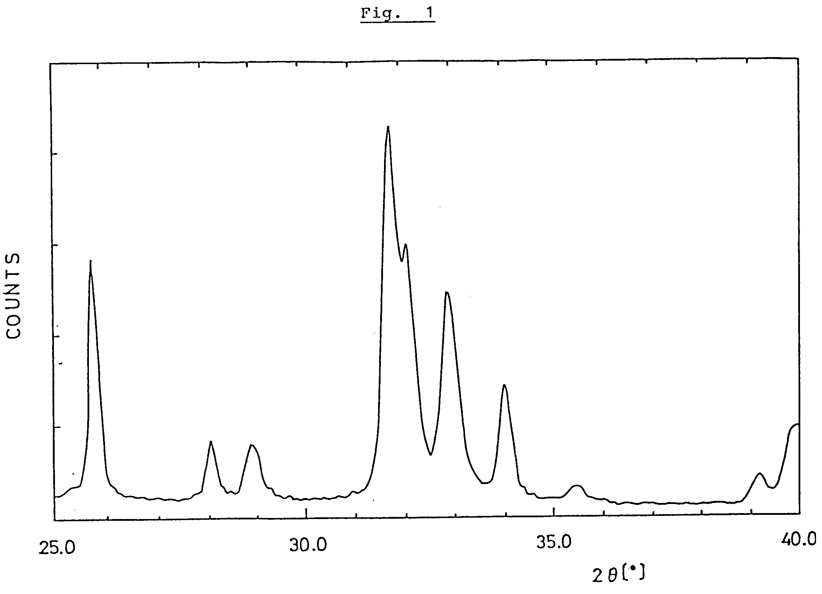

An aquous solution of phosphoric acid was reacted with a suspension of calcium hydroxide by a conventional method, and the reaction product was dried to obtain hydroxyapatite. The identity of the product was established by X-ray diffraction, the results of which are shown by an X-ray diffraction scan in Fig. 1. A chemical analysis revealed that the resulting hydroxyapatite had a Ca/P molar ratio of 1.67.

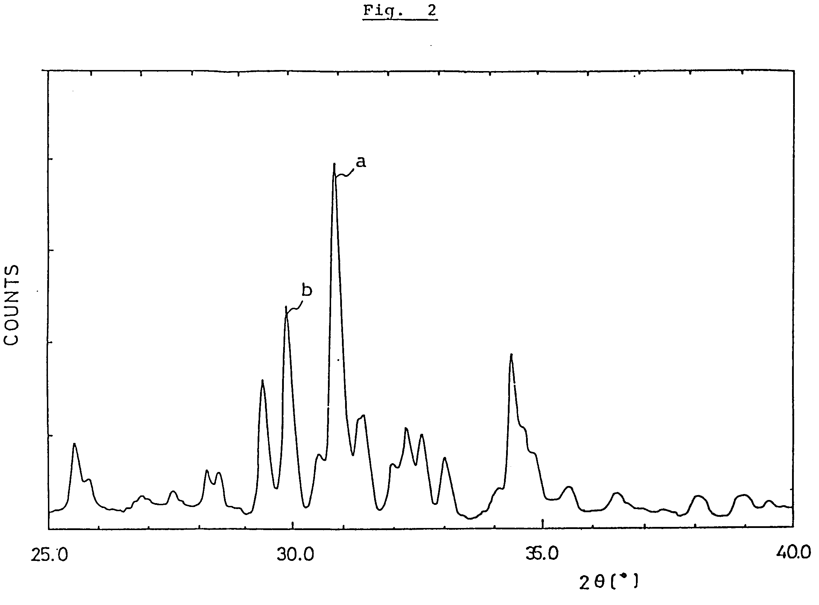

This hydroxyapatite was calcined at 1,200°C for 1 hour at a pressure of 1.3 × 10⁻⁴ Pa. The resulting product was analyzed by X-ray diffraction as above and the results are shown in Fig. 2. The appearance of two characteristic peaks a (α-tricalcium phosphate) and b (tetracalcium phosphate) verified the production of a mixture of α-tricalcium phosphate and tetracalcium phosphate by thermal decomposition of hydroxyapatite.

Three grams of this mixture in powder form was mixed with 2 g of a commercial aqueous solution of gluconic acid (product of Wako Pure Chemical Industries, Ltd.; concentration, ca. 50%). The mixture underwent a hydrolytic reaction and hardened in about 1 hour.

### EXAMPLE 2

The powder prepared as in Example 1 was mixed with hardening solutions (indicated in Table 1) at a weight ratio of 1/1 and hardening materials were obtained. The hardening times and the temperatures produced by the exothermic reaction of the hardening process are also shown in Table 1.

**TABLE 1**

| Composition of Hardening Solution (wt%) | | | | Hardening time (min) | Temperature (°C) |
|---|---|---|---|---|---|
| Citric acid | Glycerin | Sorbitol | Saccharose | | |
| 45 | 10 | 0 | 0 | 0.75 | 55 |
| 45 | 20 | 0 | 0 | 2 | 51 |
| 45 | 30 | 0 | 0 | 8 | 45 |
| 45 | 0 | 10 | 0 | 0.8 | 53 |
| 45 | 0 | 20 | 0 | 2 | 51 |
| 45 | 0 | 0 | 10 | 1 | 52 |
| 45 | 0 | 0 | 20 | 2.8 | 49 |
| 45 | 0 | 0 | 0 | 0.5 | 66 |

### EXAMPLE 3

The mixture of α-tricalcium phosphate and tetracalcium phosphate prepared in Example 1 was used as a powder component. Two grams of this powder was mixed with 1 g of a hardening solution prepared by dissolving 0.1 g of chitosan ("Flownak N" of Kyowa Yushi Kogyo K.K.) in 10 g of an aqueous solution of 40% citric acid. A plastic gum-like composition was obtained and this composition hardened in about 2 minutes to produce a hardening material having a high strength.

### COMPARATIVE EXAMPLE 1

The same procedures as in Example 3 were repeated except that chitosan was not added to the hardening solution. The composition obtained was not plastic and had a deteriorated formability although the composition hardened in about 2 minutes.

### EXAMPLE 4

The mixture of α-tricalcium phosphate and tetracalcium phosphate prepared in Example 1 was used as a powder component. Two grams of this powder was mixed with 1 g of a hardening solution prepared by dissolving 3 g of saccharose and 0.1 g of chitosan ("Flownak N" of Kyowa Yushi Kogyo K.K.) in 10 g of an aqueous solution of 40% citric acid. A plastic gum-like composition was obtained and this composition hardened in about 9 minutes to produce a hardening material having a high strength.

### COMPARATIVE EXAMPLE 2

The same procedures as in Example 4 were repeated except that chitosan was not added to the hardening solution. The composition obtained was not plastic and had a deteriorated formability although the composition hardened in about 2 minutes.

The α-tricalcium phosphate and tetracalcium phosphate produced by the process of the invention are used as a powder and mixed with a hardening solution which is an aqueous acidic solution having a polysaccharide dissolved therein. The resulting composition is highly plastic and can be readily formed into a desired shape before hardening. The hardening reaction which occurs is mild and it proceeds at a moderate rate and uniformly to produce a hardening material having a high strength and which yet will not harm body tissues.

An aqueous acidic solution containing not only a polysaccharide but also at least one of glycerin, a monosaccharide, an oligosaccharide and a sugaralcohol may be used as a hardening solution. This offers further advantage of allowing the hardening reaction to proceed more uniformly to a fuller extent at a moderate and mild rate, thereby producing a hardening material having a high strength and which yet will not harm body tissues.

## Claims

1. A process for producing a calcium phosphate type powder comprising the step of calcining, at a temperature of from about 1,150°C to 1,450°C under reduced pressure, a hydroxyapatite having a molar ratio of Ca/P of about 1.8 or less and more than 1.5 so as to produce a mixture of α-tricalcium phosphate and tetracalcium phosphate.

## Patentansprüche

1. Verfahren zur Herstellung eines Pulvers vom Kalziumphosphattyp, umfassend den Schritt der Kalzinierung eines Hydroxylapatits mit einem Molverhältnis von Ca/P von ca. 1,8 oder weniger und mehr als 1,5 bei einer Temperatur von ca. 1150°C bis 1450°C unter vermindertem Druck, so daß eine Mischung von α-Trikalziumphosphat und Tetrakalziumphosphat hergestellt wird.

## Revendications

1. Procédé de production d'une poudre de type phosphate de calcium comprenant l'étape consistant à calciner, à une température comprise entre environ 1 150°C et 1 450°C, sous pression réduite, une hydroxyapatite ayant un rapport molaire de Ca/P inférieur ou égal à 1,8 environ et supérieur à 1,5, de façon à produire un mélange de phosphate d'α-tricalcium et de phosphate de tétracalcium.
